## Europäisches Patentamt
## European Patent Office
## Office européen des brevets

(11) Veröffentlichungsnummer: **0 138 088**
**B1**

# EUROPÄISCHE PATENTSCHRIFT

(45) Veröffentlichungstag der Patentschrift:
**10.12.86**

(21) Anmeldenummer: **84111156.0**

(22) Anmeldetag: **19.09.84**

(51) Int. Cl.⁴: **A 61 N 5/10**, **A 61 N 5/01**,
**G 21 F 5/00**, **G 21 K 5/00**

(54) **Strahlenbehandlungsgerät.**

(30) Priorität: **30.09.83 DE 3335438**

(43) Veröffentlichungstag der Anmeldung:
**24.04.85 Patentblatt 85/17**

(45) Bekanntmachung des Hinweises auf die Patenterteilung:
**10.12.86 Patentblatt 86/50**

(84) Benannte Vertragsstaaten:
**BE DE FR GB NL**

(56) Entgegenhaltungen:
**DD - A - 146 518**
**FR - A - 2 060 453**
**FR - A - 2 207 734**
**US - A - 3 861 380**

(73) Patentinhaber: **Sauerwein, Kurt, Dr., Bergische
Strasse 16, D-5657 Haan 1 (DE)**

(72) Erfinder: **Kinzer, Norbert, Dipl.-Ing., Am
Kohlenmeiler 155, D-5600 Wuppertal 22 (DE)**
Erfinder: **Weinlich, Karl, Dipl.-Ing., Weberstrasse 18,
D-5600 Wuppertal 2 (DE)**
Erfinder: **Sauerwein, Kurt, Dr., Kattendahl 7,
D-4006 Erkrath (DE)**

(74) Vertreter: **Schumacher, Horst, Dr. Dipl.-Phys.,
Frühlingstrasse 43 (Ecke Holunderweg),
D-4300 Essen 1 (DE)**

ACTORUM AG

# Beschreibung

Die Erfindung betrifft ein Strahlenbehandlungsgerät gemäss den Oberbegriffen von Patentansprüchen 1 und 6.

Derartige Strahlenbehandlungsgeräte finden vor allem in der medizinischen Strahlenbehandlung, insbesondere bei der Behandlung von z.B. Tumoren Anwendung. Dort werden als Hohlsonden sogenannte Applikatoren, d.h. dünne Hohlnadeln oder Kunststoffflansche in das Zielobjekt (Tumor) implantiert. Nachfolgend wird ein Strahlerhalter, z.B. eine gewickelte Feder mit Abstandshaltern und einer oder mehreren strahlungsaktiven Zonen in die Hohlsonde ein- und damit an den Behandlungsort herangeführt (afterloading). Während des Nichtgebrauchs der Strahler müssen diese in einem Strahlenschutzbehälter aufbewahrt werden. Während des Überführens der Strahler von dem Strahlenschutzbehälter in die Hohlsonde ist das Bedienpersonal der — wenn auch nicht allzu starken — radioaktiven Strahlung der Strahler ausgesetzt. Dieser Nachteil beim Vonhandeinsetzen des Strahlerhalters in die Hohlsonde ist bei einem automatisch arbeitenden afterloading-Gerät der eingangs genannten Art behoben worden, bei dem der Strahlenschutzbehälter einen einzigen Strahlerhalter aufnimmt, welcher mittels einer Rohrkanalweiche an der Ausgangsseite des Strahlenschutzbehälters in einen vorgebbaren, mit der Hohlsonde im Durchgangssinne verbundenen Schlauch ein- und ausfahrbar ist.

Dieses bekannte Strahlenbehandlungsgerät weist den Nachteil auf, dass nur ein einziger Strahlerhalter mit einer darauf befindlichen einzigen Strahlenquelle verwendet werden kann, welche innerhalb der Hohlsonde von Behandlungsort zu Behandlungsort verfahren werden und nach Abschluss der Behandlung in dieser Hohlsonde in den Strahlenschutzbehälter zurück- und nach Verstellen der Rohrkanalweiche in die nächste Hohlsonde eingefahren werden muss. Aus dieser zwangsläufig vorgegebenen Vorgehensweise ergibt sich eine sehr lange Behandlungsdauer, während der der zu behandelnde Gegenstand oder Patient an das Strahlenbehandlungsgerät angeschlossen sein muss. Insbesondere ist es nicht möglich, verschiedene Strahlerhalter einzusetzen, so dass in den einzelnen Hohlsonden nach Bedarf auch zwei oder mehr vorgebbar beabstandete Strahler auf einem Strahlerhalter verwendbar wären. In der Regel müssen nämlich in den verschiedenen Hohlsonden am Behandlungsort unterschiedlich viele und unterschiedlich beabstandete Strahlungspunkte realisiert werden.

Aus der US-PS 3 861 380 ist ein Strahlenbehandlungsgerät der eingangs genannten Art bekannt, bei dem zum Ein- und Ausfahren jeden einzelnen Strahlerhalters eine gesonderte Antriebsvorrichtung erforderlich ist — die übrigen Strahler verbleiben also im Strahlenschutzbehälter; diese Fahrweise ist nur bei voneinander unabhängigen Antrieben der einzelnen Strahlerhalter möglich. Bei diesem bekannten Strahlenbehandlungsgerät ist die für das Bedienpersonal und den Patienten erforderliche Strahlensicherheit offensichtlich nur dann zu gewährleisten, wenn vor jeder Strahlenbehandlung eine Funktionsprüfung des Gerätes ohne Strahler durchgeführt wird. Es ist verständlich, dass die getrennten Antriebe der einzelnen Strahler einen erheblichen Raum in Anspruch nehmen, so dass dieses bekannte Strahlenbehandlungsgerät nur mit vergleichsweise wenigen getrennt arbeitenden Strahlern ausrüstbar ist (das Gerät muss ja hinreichend klein bleiben, um fahrbar zu sein, so dass die erwähnten Testläufe in den hierfür vorgesehenen Räumen vorgenommen werden können).

Davon ausgehend liegt der Erfindung die Aufgabe zugrunde, ein Strahlenbehandlungsgerät der eingangs genannten Art zu schaffen, bei dem aus der Vielzahl der Strahlerhalter, die mit unterschiedlich vielen und/oder unterschiedlich weit beabstandeten Strahlungsquellen ausgerüstet sein können, eine vorgebbare Anzahl von Strahlerhaltern gleichzeitig mittels einer, insbesondere einzigen Antriebsvorrichtung in die entsprechende Anzahl von Hohlsonden einführbar ist, wobei die Strahlenbelastung des Bedienungspersonals stets, insbesondere auch während der Strahlenbehandlung, auf ein unabwendbares Minimum herabgesetzt sein sollen.

Als technische Lösung wird hierfür ein Strahlenbehandlungsgerät der eingangs genannten Art gemäss den kennzeichnenden Merkmalen von Patentanspruch 1 oder, als alternative Lösung, gemäss den kennzeichnenden Merkmalen von Patentanspruch 6 vorgeschlagen. Bei einer praktischen Ausführungsform der Erfindung ist der (sind die) Strahlenschutzbehälter mit Führungsrohren derart verbunden, dass die Antriebskabel — von der Antriebseinheit ausgehend — durch die Führungsrohre und nachfolgend durch den (die) Strahlenschutzbehälter sowie die nachfolgenden flexiblen Schläuche axial geführt verfahrbar sind. Hierdurch wird eine besonders gute Führung der Antriebskabel erreicht und der erste Strahlenschutzbehälter (gemäss Anspruch 1) kann besonders kleinvolumig gehalten werden, da die Antriebskabel sehr dicht nebeneinander liegen können.

Mit der Erfindung wird u.a. der Vorteil erreicht, dass jede beliebige Kombination von den in dem Strahlenbehandlungsgerät vorgesehenen Strahlerhaltern gleichzeitig an den Behandlungsort herangefahren werden kann, so dass die Strahlenbehandlung gleichzeitig an allen erforderlichen Strahlenbehandlungspunkten des Zielobjektes durchgeführt werden kann. Hierzu ist grundsätzlich nur eine einzige Antriebseinheit erforderlich, weil — gemäss Anspruch 1 — die jeweils nicht benötigten Strahlerhalter durch ihre zugeordneten flexiblen Schläuche in den weiteren Strahlenschutzbehälter geführt werden, während die jeweils benötigten Strahlerhalter in die zugeordneten Hohlsonden geführt werden, so dass während der Behandlungsdauer auch die nicht benötigten Strahlungsquellen abgeschirmt gelagert sind und sich die Strahlenbelastung der Umwelt auf die kurze Zeitspanne reduziert, in der die Strahlerhalter vom Ausgang des ersten Strahlenschutzbehälters durch den flexiblen Schlauch in die Hohlsonde bzw. den weiteren Strahlenschutzbehälter einfahren – bzw. umgekehrt.

Bei dem alternativ vorgeschlagenen Strahlenbehandlungsgerät gemäss Anspruch 6 können die freien Enden der Schläuche in gleicher Weise mit Schlauchkupplungselementen ausgestattet sein wie im Merkmal c) des Anspruchs 1. Auf solche Schlauchkupplungen kann dann verzichtet werden, wenn die Hohlsonden mit den Schlauchenden fest verbunden sind und daher einen Bestandteil des erfindungsgemässen Kassettenelementes bilden. Ebenso ist es bei dieser Erfindungsalternative möglich, die Schläuche mit entsprechenden Schlauchkupplungen lösbar an dem Strahlenschutzbehälter des Kassettenelementes anzuordnen. Die Unterbringung des Antriebskabels kann alternativ entweder mittels einer an dem Kassettengehäuse drehbar, insbesondere axial verschieblich, angeordneten Windentrommel, auf die das Antriebskabel spiralig aufwickelbar ist, erfolgen oder mittels eines drehbaren oder feststehenden, an dem Kassettengehäuse angeordnetes Gehäuse mit einem scheibenförmigen Hohlraum mit einer Durchtrittsöffnung für das Antriebskabel in einer Stirnfläche, wobei die Hohlraumweite etwa der Dicke des Antriebskabels entspricht und das Antriebskabel durch Einschieben spiralförmig in das Gehäuse einlegbar ist.

Eine die Antriebskabel ihrer gesamten Vortriebslänge nach aufnehmende Antriebstrommel gemäss Anspruch 2 gestattet eine einwandfreie Führung der Antriebskabel sowie deren platzsparende Anordnung.

Wenn die Antriebskabel im Bereich zwischen der Antriebseinheit und dem (den) Strahlenschutzbehälter(n) zwischen Führungsrillen einer Antriebs- oder Windentrommel und einem in Antriebsrichtung verfahrbaren Abdeckband angeordnet sind, werden sie ohne Reibungsverluste über die gesamte Vortriebslänge sicher geführt und können vergleichsweise geringe Abmessungen und Biegesteifigkeit aufweisen, was wiederum der erforderlichen Flexibilität beim Verfahren innerhalb der flexiblen Schläuche zugutekommt.

Wenn die antriebsseitigen Enden der Antriebskabel axial-federnd in einer Schalterleiste angeordnet sind, können vor, nach und während dem Verfahren der Strahlerhalter elektrische Schaltimpulse verschiedene Kontroll- und Warnfunktionen ausüben. Wenn z.B. ein Strahler während des Verfahrens auf einen Widerstand stösst, der ein Weiterfahren nicht gestattet, so löst das entsprechende Antriebskabel einen Schaltimpuls und damit eine Fehleranzeige für den betreffenden Strahlerhalter aus. Dies ist vor allem dann von Vorteil, wenn die schlauchseitigen Schlauchkupplungselemente eine Sperrklinke aufweisen, die den freien Durchgang verlegen, sobald das betreffende Schlauchende nicht mit einer Hohlsonde oder dem weiteren Strahlenschutzbehälter ordnungsgemäss zusammengekuppelt ist; ohne eine solche Sperrklinke und Fehlermeldung könnte nämlich ein Strahlerhalter ansonsten unbemerkt ohne jeglichen Strahlenschutz an die Umgebungsatmosphäre gelangen. Des weiteren gestattet die erfindungsgemässe Schalterleiste eine Kontaktgabe beim Erreichen der Endlagen der Strahlerhalter in den Hohlsonden oder dem weiteren Strahlenschutzbehälter. Hierbei kann auch angezeigt werden, welche Strahlerhalter in die Hohlsonden und welche Strahlerhalter in den Strahlenschutzbehälter eingefahren sind. Letzteres wird bevorzugt dadurch erreicht, dass sich die Gesamtlänge jedes Schlauches und der damit zu verbindenden Hohlsonde von der Gesamtlänge desselben Schlauches und des damit zu verbindenden freien Raumes des weiteren Strahlenschutzbehälters um mindestens den Schaltweg eines Endschalters (in der Schaltleiste) unterscheidet.

Die bestmögliche Handhabe der Schlauchkupplungselemente bei geringstmöglichen Abmessungen der Strahlenschutzbehälter wird erfingungsgemäss dadurch erreicht, dass die korrespondierenden Schlauchkupplungselemente des weiteren Strahlenschutzbehälters weiter als die aus dem anderen Strahlenschutzbehälter austretenden flexiblen Schläuche beabstandet sind.

Die vorgenannten, erfindungsgemäss zu verwendenden Bauteile unterliegen in ihrer Grösse, Formgestaltung, Materialauswahl und technischen Konzeption keinen besonderen Ausnahmebedingungen, so dass die in dem jeweiligen Anwendungsgebiet bekannten Auswahlkriterien uneingeschränkt Anwendung finden können.

Weitere Einzelheiten, Merkmale und Vorteile des Gegenstandes der Erfindung ergeben sich aus der nachfolgenden Beschreibung der zugehörigen Zeichnung, in der eine bevorzugte Ausführungsform eines erfindungsgemässen Strahlenbehandlungsgerätes dargestellt worden ist. In der Zeichnung zeigen:

Fig. 1 ein Strahlenbehandlungsgerät in Seitenansicht;

Fig. 2 dasselbe Strahlenbehandlungsgerät in Ansicht A gemäss Fig. 1;

Fig. 3 eine Antriebstrommel in radialer Ansicht – z.T. im Schnitt;

Fig. 4 dieselbe Antriebstrommel in Axialansicht – entsprechend der Fig. 1;

Fig. 5 dieselbe Antriebstrommel im vergrösserten Radialschnitt;

Fig. 6 einen Strahlenschutzbehälter mit Eingangs- und Ausgangsöffnung;

Fig. 7 einen weiteren Strahlenschutzbehälter nur mit Eingangsöffnung;

Fig. 8 ein anderes Strahlenbehandlungsgerät in Ansicht von oben (Ansicht B gemäss Fig. 9);

Fig. 9 dasselbe Strahlenbehandlungsgerät im Schnitt entlang der Linie IX-IX gemäss Fig. 8;

Fig. 10 dasselbe Strahlenbehandlungsgerät im Schnitt entlang der Linie X-X gemäss Fig. 9 – im vergrösserten Ausschnitt;

Fig. 11 eine Kassetteneinheit für ein ähnliches Strahlenbehandlungsgerät wie in Fig. 8 sowie

Fig. 12 dieselbe Kassetteneinheit in Ansicht C gemäss Fig. 11.

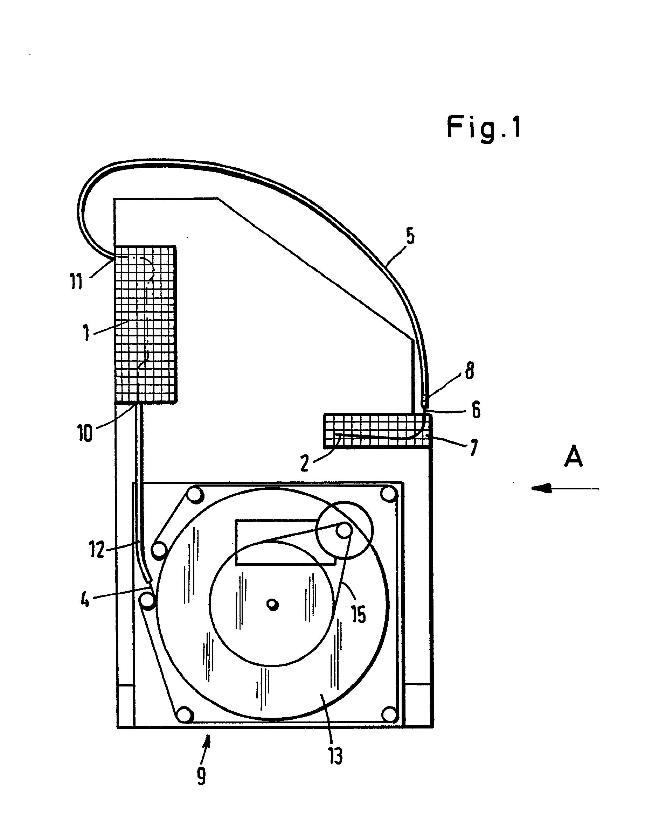

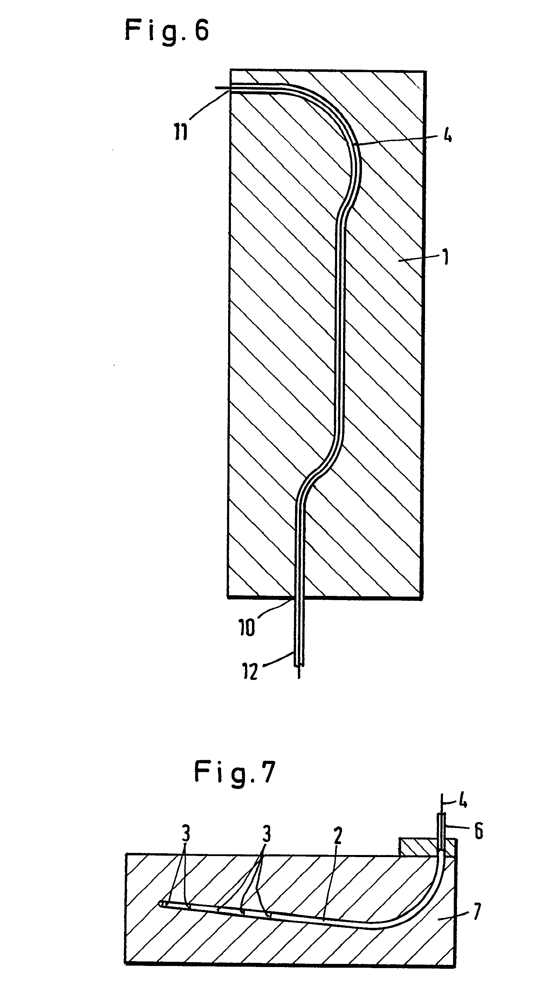

Das Strahlenbehandlungsgerät gemäss Fig. 1 besteht aus einem ersten Strahlenschutzbehälter 1 (Ruhebehälter), einer Vielzahl von z.B. 48 Strahlerhaltern 2 (Fig. 7) mit darauf zwischen Abstandshaltern unterschiedlich weit beabstandeten, ein oder mehreren an sich bekannten radioaktiven Strahlungsquellen 3. Diese Strahlerhalter sind an dem einen freien Ende je eines flexiblen Antriebskabels 4 angeordnet, welches über möglichst lange Strecken geführt ist und den auftretenden Druck- und Zugbelastungen ohne Ausknikkungen standhält. Von dem Strahlenschutzbehälter 1 führen der Anzahl der Strahlerhalter entsprechend viele Ausgangsöffnungen in damit verbundene flexible Schläuche 5. Diese tragen an ihren anderen freien Enden je ein — in der Zeichnung nicht eigens dargestelltes, da an sich bekanntes — Schlauchkupplungselement zum Verbinden des freien Schlauchendes mit einer ebenfalls nicht dargestellten, da an sich bekannten, Hohlsonde mit einem korrespondierenden Schlauchkupplungselement. Dieses korrespondierende Schlauchkupplungselement entspricht weiteren korrespondierenden Schlauchkupplungselementen 6 an den der Anzahl der Strahlerhalter entsprechenden Eingangsöffnungen eines weiteren Strahlenschutzbehälters 7 (Zwischenbehälter), der alle Strahlerhalter gemeinsam und vollständig aufnehmen kann. Die freien Schlauchenden 8 können somit wahlweise mit dem Strahlenschutzbehälter 7 oder einer Hohlsonde am Messort lösbar verbunden werden und Durchgangsverbindungen für die Strahlerhalter und die Enden der Antriebskabel herstellen.

Alle Antriebskabel 4 weisen eine gemeinsame Antriebseinheit 9 auf, die von einer einzigen Steuereinheit gesteuert wird.

Der erste Strahlenschutzbehälter 1 weist der Anzahl der Strahlerhalter entsprechend viele Eingangsöffnungen 10 und Ausgangsöffnungen 11 auf, zwischen denen zumindest eine Teilstrecke mit S-förmig gebogenen Führungsrohren für die Strahlerhalter und insbesondere die Antriebskabel 4 versehen sein kann. Weitere Führungsrohre 12 verbinden die Eingangsöffnungen 10 des Strahlenschutzbehälters 1 mit der Antriebseinheit 9.

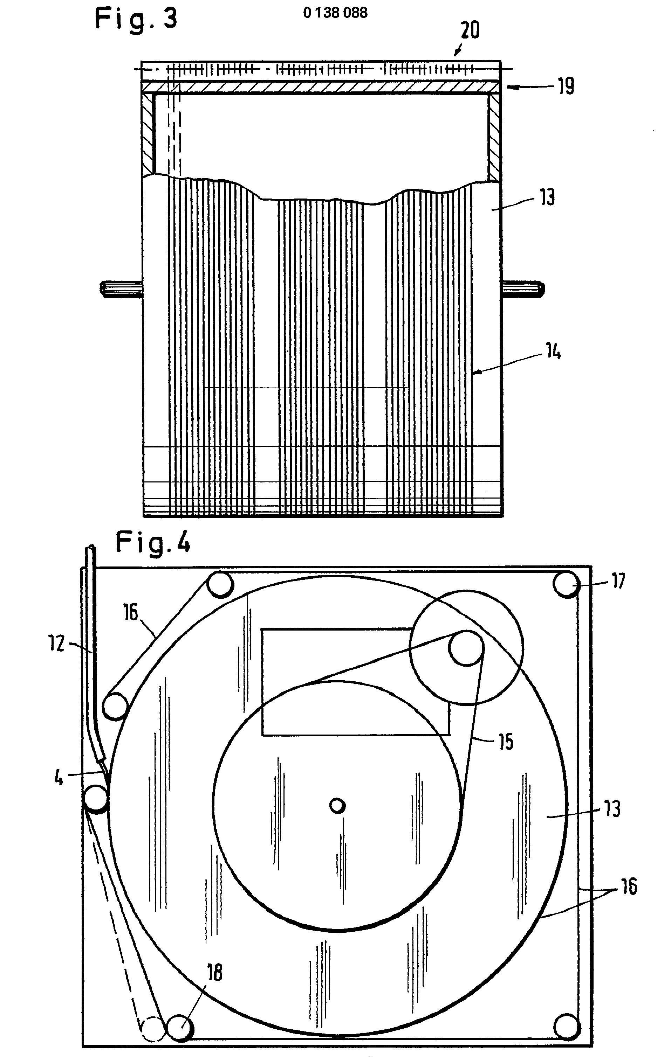

Die Antriebseinheit 9 besteht im wesentlichen aus einer Antriebstrommel 13, deren Umfang grösser als die maximale Vortriebslänge ist und radiale Führungsrillen 14 für die einzelnen Antriebskabel 4 aufweist (Fig. 3). Die Antriebstrommel kann mit einem bekannten Riementrieb 15 vorwärts und rückwärts gedreht werden (Fig. 4).

Ein Abdeckband 16 mit der Breite der Antriebstrommel 13 ist derart endlos mittels Umlenkrollen 17 und einer Spannrolle 18 um den Zylindermantel der Antriebstrommel 13 geführt, dass die Antriebskabel 4 in den Führungsrillen 14 auch bei der Drehbewegung ruhen und nicht ausknicken können. Das Abdeckband 16 umspannt den Trommelumfang bis auf ein sehr kurzes Umfangstück, in dessen Bereich die Antriebskabel 4 von den Führungsrillen 14 abheben und in die freien Enden der Führungsrohre 12 hineingleiten. Im Umfang der Antriebstrommel 13 befindet sich eine axiale Ausnehmung 19 zur Aufnahme einer axial ausgerichteten Schalterleiste 20 mit so vielen Annäherungs- oder Berührungsschaltern 21 wie Antriebskabel 4 vorgesehen sind. Die freien Enden dieser Antriebskabel sind axial-federnd in der Schalterleiste 2 befestigt und wirken mit ihren axial beweglichen Auslösern 22 auf die Schalter 21 (Fig. 5).

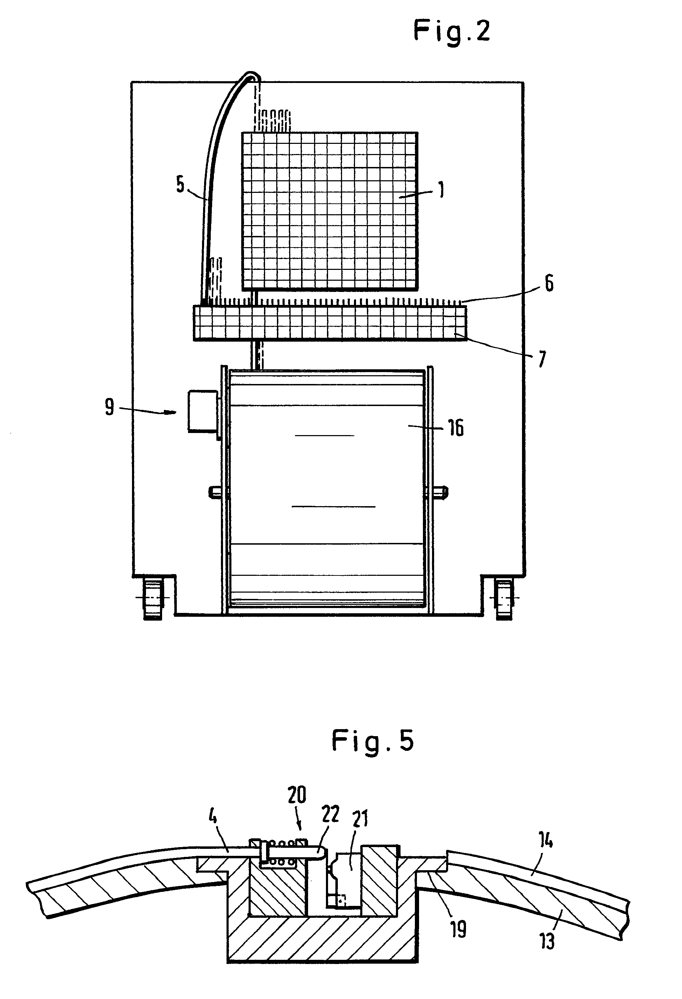

Wie sich aus der Fig. 2 ergibt, sind die korrespondierenden Schlauchkupplungselemente 6 des weiteren Strahlenschutzbehälters 7 an diesem nebeneinander derart weit beabstandet, dass eine akzeptable Handhabe der Kupplungselemente gegeben ist, während die flexiblen Schläuche 5 und die Ausgangsöffnungen 11 an dem ersten Strahlenschutzbehälter 1 weniger weit beabstandet sind. Die Schläuche 5 sind in Fig. 2 der Übersicht halber nur teilweise dargestellt worden.

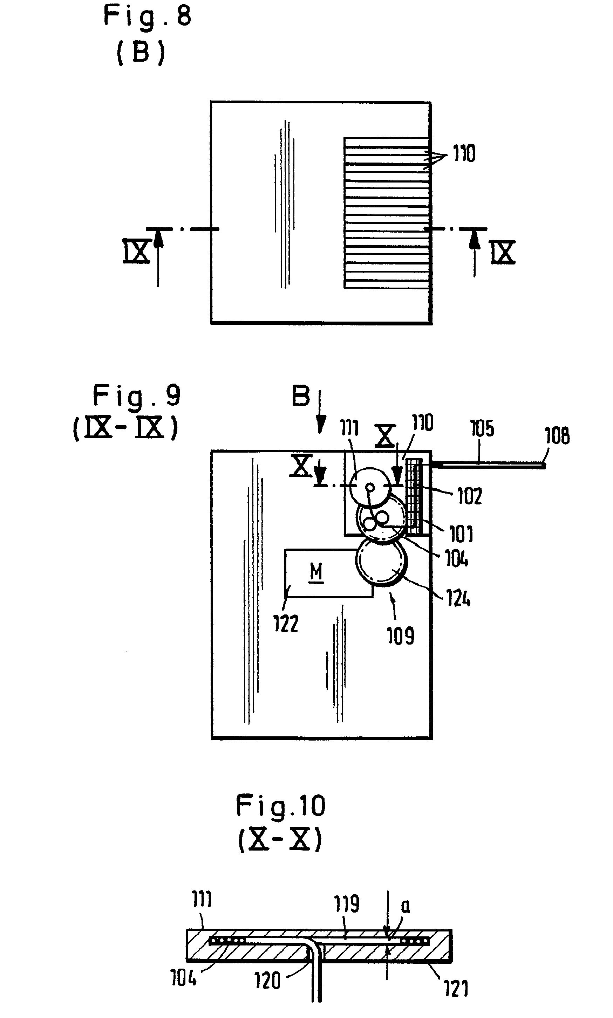

In das in Fig. 8 nur schematisch dargestellte Strahlenbehandlungsgerät sind von oben und/oder der Seite her eine bestimmte Vielzahl von Kassettengehäusen 110 nebeneinander lösbar einsetzbar. Durch die Geräteabmessungen ist die maximale Anzahl von nebeneinander darin einsetzbaren Kassettengehäusen festgelegt. Im Normalfall werden aber weniger als die maximal mögliche Anzahl an Kassettengehäusen eingeschoben sein, nämlich nur soviele Kassetten wie Strahler erforderlich sind; die übrigen Kassettenplätze bleiben unbesetzt.

Ein Kassettengehäuse 110 kann z.B. aus Kunststoff oder Metall hergestellt und mit an sich bekannten Rastelementen ausgerüstet sein, so dass ein sicheres ortsfestes Einsetzen des Kassettengehäuses in das Strahlenbehandlungsgerät möglich ist. Jede einzelne Kassetteneinheit nimmt nun in dem Kassettengehäuse 110 einen Strahlenschutzbehälter 101 und ein Antriebskabel 104 mit einem endseitig daran befestigten Strahlerhalter 102 auf. Diese drei Grundelemente entsprechen in ihrem grundsätzlichen Aufbau dem Strahlenschutzbehälter 1, dem Strahlhalter 2 und dem Antriebskabel 4 in den Figuren 1 bis 7.

Grundsätzlich ist es möglich, in jedem Kassettengehäuse 110 einen eigenen motorischen Antrieb für das Antriebskabel 104 unterzubringen, so dass lediglich die Energieversorgung durch entsprechende Steckkontakte am Kassettengehäuse beim Einsetzen des Kassettengehäuses in das Strahlenbehandlungsgerät erfolgt. Bevorzugt befindet sich aber der motorische Antrieb ausserhalb des Kassettengehäuses im Strahlenbehandlungsgerät (siehe Fig. 9), wobei entweder für jedes Kassettenelement ein einzelner Motor oder — wie bevorzugt — ein gemeinsamer Antriebsmotor 122 für alle Kassettenelemente gemeinsam vorgesehen ist.

Die Kraftübertragung des Antriebsmotors auf die Antriebskabel 104 kann über miteinander im Eingriff stehende Zahnräder, über miteinander im Reibschluss stehende Antriebswalzen oder über andere an sich bekannte Kupplungsorgane erfolgen. Dabei ist in oder an dem Kassettengehäuse 110 das jeweils eine Kupplungselement, Zahnrad o.dgl. und in dem übrigen Strahlenbehandlungsgerät das damit korrespondierende Kupplungselement, Zahnrad o.dgl. derart angeordnet, dass beim Einsetzen des Kassettenelementes in das Strahlenbehandlungsgerät die korrespondierenden Kupplungselemente wirkverbunden bzw. wirkverbindbar sind.

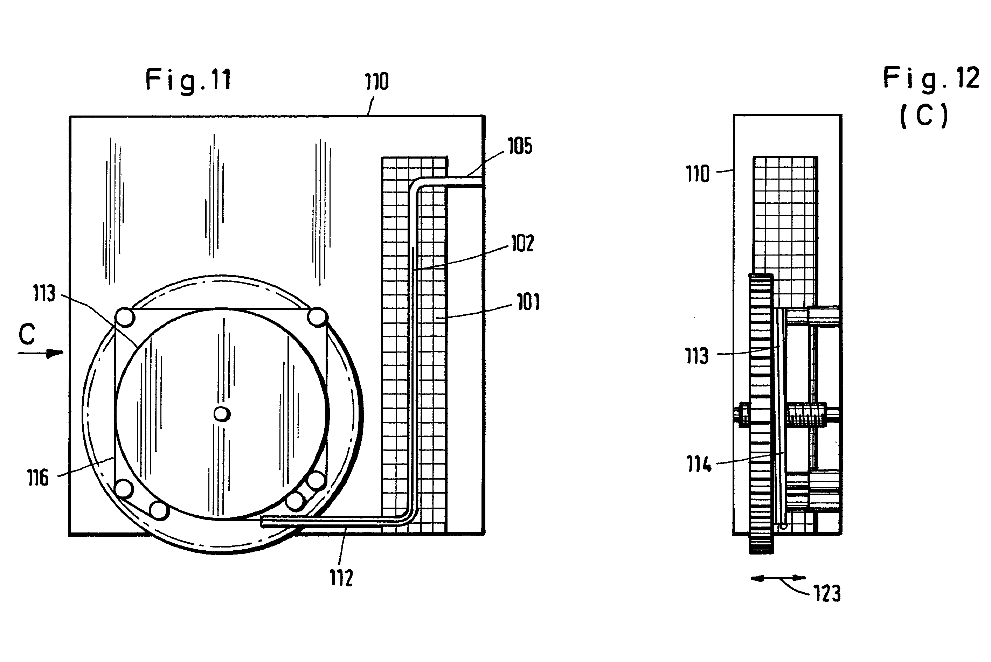

Das Antriebskabel 104 sollte innerhalb des Kassettengehäuses auf möglichst kleinem Raum unterzubringen sein. Hierzu eignet sich eine Windentrommel 113 (Fig. 11, 12), auf die der noch nicht ausgefahrene Teil des Antriebskabels spiralig aufwickelbar ist. Die Windentrommel 113 wird dann in vorbeschriebener Weise nach dem Einsetzen des Kassettengehäuses 110 in das Strahlenbehandlungsgerät angetrieben. Dabei empfiehlt es sich, eine axiale Verschieblichkeit der Windentrommel 113 gemäss dem Pfeil 123 in Fig. 12 vorzusehen, so dass das Antriebskabel ohne Biegungen in das sich anschliessende Führungsrohr 112 einschiebbar ist. Es kann eine ähnliche Abdeckung der Windentrommel 113 mit einem Abdeckband wie im Zusammenhang mit Fig. 1 bis 5 beschrieben, vorgesehen sein.

Eine andere Unterbringungsmöglichkeit für das nicht ausgefahrene Antriebskabelende besteht in einem Gehäuse 111, gemäss Fig. 9, 10, das einen bevorzugt kreisscheibenförmigen Hohlraum 119 aufweist, dessen Hohlraumweite a (lichte Weite) nur geringfügig grösser als die Dicke des Antriebskabels 104 ist. Dieses Gehäuse 111 kann — seiner Hohlraumform entsprechen — ein flacher Zylinder sein, der von einer Antriebswalze 124 angetrieben wird, wobei der Antriebsmotor 122 die Antriebswalze 124 antreibt. Diese Antriebswalze 124 kann auf die Gehäuse 111 aller in das Strahlenbehandlungsgerät eingesetzten Kassettenelemente gleichzeitig wirken. Für den Austritt des Antriebskabels 104 dient eine Durchtrittsöffnung 120 in der Mitte der Stirnfläche 121 des Gehäuses 111. Das nicht ausgefahrene Ende des Antriebskabels 104 liegt spiralförmig in dem Hohlraum 119 und ist mit seinem freien Ende dort festgelegt; somit führt ein Drehen des Gehäuses 111 in vorgeschriebener Weise zu einem Aus- oder Einfahren des Antriebskabels 104. Diese Art der Antriebskabelbetätigung ist selbstverständlich ausserordentlich vielseitig und daher nicht ausschliesslich in dem erfindungsgemässen Strahlenbehandlungsgerät einsetzbar.

Der besondere Vorteil der erfindungsgemässen Kassettenelemente besteht darin, dass die zu verwendenden Strahler vom behandelnden Personal nicht mehr gewechselt werden müssen, sie können vielmehr einen integralen Bestandteil des Kassettenelementes bilden. Bei der Anwendung des erfindungsgemässen Strahlenbehandlungsgerätes ist man also auch nicht gezwungen, mit einer einzigen Strahlerart auszukommen oder bereits beim Herstellen des Gerätes endgültig festzulegen, mit welchen Strahlern die einzelnen Strahlerhalter auszurüsten sind. Ausserdem kann eine Kupplung zwischen Strahlerhalter und Antriebskabel entfallen.

Bezugszeichenliste

1 erster Strahlenschutzbehälter (Ruhebehälter)
2 Strahlenhalter
3 Strahlungsquellen
4 Antriebskabel
5 Schläuche
6 Schlauchkupplungselement
7 weiterer Strahlenschutzbehälter (Zwischenbehälter)
8 freie Schlauchenden
9 Antriebseinheit
10 Eingangsöffnungen
11 Ausgangsöffnungen
12 Führungsrohre
13 Antriebstrommel
14 Rührungsrillen
15 Riementrieb
16 Abdeckband
17 Umlenkrollen
18 Spannrolle
19 Ausnehmung
20 Schalterleiste
21 Schalter
22 Auslöser
101 Strahlenschutzbehälter
102 Strahlerhalter
104 Antriebskabel
105 Schläuche
108 freie Schlauchenden
109 Antriebseinheit
110 Kassettengehäuse
111 Gehäuse
112 Führungsrohre
113 Windentrommel
114 Führungsrillen
116 Abdeckband
119 Hohlraum
120 Durchtrittsöffnung
121 Stirnfläche
122 Antriebsmotor
123 Pfeil
124 Antriebswalze
A Ansicht
B Ansicht
C Ansicht
a Hohlraumweite

**Patentansprüche**

1. Strahlenbehandlungsgerät, bestehend aus
a) mindestens einem Strahlenschutzbehälter (1) (Ruhebehälter),
b) daraus durch eine entsprechende Anzahl damit verbundener flexibler Schläuche (5) mittels einer entsprechenden Anzahl von Antriebskabeln (4) axial ein- und ausfahrbaren Strahlerhaltern (2) und
c) einer entsprechenden Anzahl von Schlauchkupplungselementen an den freien Enden (8) der Schläuche (5) zum Herstellen lösbarer Durchgangsverbindungen zu einer entsprechenden Anzahl von Hohlsonden, dadurch gekennzeichnet, dass
d) alle Antriebskabel (4) mit einer gemeinsamen Antriebseinheit (9) im Ein- und Ausfahrsinne verbunden sind und

e) ein weiterer Strahlenschutzbehälter (7) (Zwischenbehälter) mit einer gleich grossen Vielzahl korrespondierender Schlauchkupplungselemente (6) zum Herstellen von Durchgangsverbindungen zu den flexiblen Schläuchen (5) vorgesehen und derart angeordnet ist, dass alle flexiblen Schläuche (5) mit ihm lösbar verbindbar sind.

2. Strahlenbehandlungsgerät nach Anspruch 1, gekennzeichnet durch eine an sich bekannte Antriebstrommel (13), deren Umfang grösser als die maximale Vortriebslänge ist.

3. Strahlenbehandlungsgerät nach Anspruch 2, dadurch gekennzeichnet, dass die antriebsseitigen Enden (Auslöser 22) der Antriebskabel (4) axialfedernd in einer Schalterleiste (20) angeordnet sind.

4. Strahlenbehandlungsgerät nach einem der Ansprüche 1 bis 3, dadurch gekennzeichnet, dass sich die Gesamtlänge jedes Schlauches (5) und der damit zu verbindenden Hohlsonde von der Gesamtlänge desselben Schlauches (5) und des damit zu verbindenden freien Raumes des weiteren Strahlenschutzbehälters (7) um mindestens den Schaltweg eines Endschalters unterscheidet.

5. Strahlenbehandlungsgerät nach einem der Ansprüche 1 bis 4, dadurch gekennzeichnet, dass die korrespondierenden Schlauchkupplungselemente (6) des weiteren Strahlenschutzbehälters (7) weiter als die aus dem anderen Strahlenschutzbehälter (1) austretenden flexiblen Schläuche (5) beabstandet sind.

6. Strahlenbehandlungsgerät, bestehend aus
a) mindestens einem Strahlenschutzbehälter (101),
b) daraus durch eine entsprechende Anzahl damit verbundener flexibler Schläuche (105) mittels einer entsprechenden Anzahl von Antriebskabeln (104) axial ein- und ausfahrbaren Strahlerhaltern (102) dadurch gekennzeichnet, dass
c) je ein Antriebskabel (104) mit einem Strahlerhalter (102) und einem Strahlenschutzbehälter (101) in einem Kassettengehäuse (110) angeordnet sind und
d) eine frei wählbare Anzahl von Kassettengehäusen (110) in das Strahlenbehandlungsgerät derart lösbar einsetzbar ist, dass die Antriebskabel (104) der eingesetzten Kassettengehäuse (110) mit einer gemeinsamen Antriebseinheit (109) des Strahlenbehandlungsgerätes antriebsverbunden sind.

7. Strahlenbehandlungsgerät nach Anspruch 6, gekennzeichnet durch eine an dem Kassettengehäuse (110) drehbar angeordnete Windentrommel (113), auf die das Antriebskabel (104) spiralig aufwickelbar ist.

8. Strahlenbehandlungsgerät nach Anspruch 6, gekennzeichnet durch ein an dem Kassettengehäuse (110) angeordnetes Gehäuse (111) mit einem Scheibenförmigen Hohlraum (119) mit einer Durchtrittsöffnung (120) für das Antriebskabel (104) in einer Stirnfläche (121), dessen Hohlraumseite (a) etwa der Dicke des Antriebskabels (104) entspricht und in welches das Antriebskabel (104) durch Einschieben spiralförmig einlegbar ist.

9. Strahlenbehandlungsgerät nach Anspruch 2 oder 7, dadurch gekennzeichnet, dass die Antriebskabel (4; 104) im Bereich zwischen der Antriebseinheit (9; 109) und dem (den) Strahlenschutzbehälter (n) (1; 101) zwischen Führungsrillen (14; 114) der Antriebs- oder Windentrommel (13 bzw. 113) und einem in Antriebsrichtung verfahrbaren Abdeckband (16; 116) angeordnet sind.

10. Strahlenbehandlungsgerät nach einem der Ansprüche 1 bis 9, dadurch gekennzeichnet, dass der (die) Strahlenschutzbehälter (1; 101) mit Führungsrohren (12; 112) derart verbunden ist (sind), dass die Antriebskabel (4; 104) durch die Führungsrohre (12; 112) und nachfolgend durch den (die) Strahlenschutzbehälter (1; 101) und die nachfolgenden flexiblen Schläuche (5; 105) axial geführt verfahrbar sind.

**Claims**

1. Radiation therapy device consisting of:
a) at least one radiation protection container (1) (resting container),
b) emitter holders (2) which can be axially inserted and pulled out therefrom by means of a corresponding number of drive cables (4), through a corresponding number of flexible tubes (5) connected thereto, and
c) a corresponding number of tube connecting elements at the free ends (8) of the tubes (5) for establishing detachable through-connections with a corresponding number of hollow probes, characterized in that
d) all the drive cables (4) are connected to a common drive unit (9) in the direction in which insertion and extraction occur and
e) an additional radiation protection container (7) (intermediate container) with an equivalent number of corresponding tube connecting elements (6) for establishing through-connections with the flexible tubes (5) is provided and arranged in such a way that all the flexible tubes (5) can be connected thereto in a detachable manner.

2. Radiation therapy device according to Claim 1, characterized by a drive drum (13) which is known per se and the circumference of which is greater than the maximum distance advanced.

3. Radiation therapy device according to Claim 2, characterized in that the ends of the drive cable (4) located on the drive side (tripping device 22) are arranged in an axially sprung manner inside a switching strip (20).

4. Radiation therapy device according to Claims 1 to 3, characterized in that the overall length of each differs from the overall length of the same tube (5) and the free space of the additional radiation protection container (7) to be connected thereto, by at least the switching path of the limit switch.

5. Radiation therapy device according to one of Claims 1 to 4, characterized in that the corresponding flexible tube connecting elements (6) of the additional radiation protection container. (7) are situated further apart than the flexible tubes (5) emerging from the other radiation protection container (1).

6. Radiation therapy device, consisting of:

a) at least one radiation protection container (101),

b) emitter holders (102) which can be axially inserted and pulled out therefrom by means of a corresponding number of drive cables (104), through a corresponding number of flexible tubes (105) connected thereto, characterized in that

c) in each case a drive cable (104) with an emitter holder (102) and a radiation protection container (101) is arranged inside a cartridge housing (110) and

d) a freely selectable number of cartridge housings (110) can be detachably inserted into the radiation therapy device in such a way that the drive cable (104) of the inserted cartridge housing (110) forms a driving connection with the drive unit (109) of the radiation therapy device.

7. Radiation therapy device according to Claim 6, characterized by a winding drum (113) which is rotatably arranged on the cartridge housing (113) and onto which the drive cable (104) can be wound in spiral fashion.

8. Radiation therapy device according to Claim 6, characterized by a housing (111) which is arranged on the cartridge housing (110) and which has a disk-shaped cavity (119) with an opening (120) in one end face (121) for allowing the drive cable to pass through, the cavity wall (a) of which corresponds approximately to the thickness of the drive cable (104) and inside which the drive cable (104) can be pushed and arranged in spiral fashion.

9. Radiation therapy device according to Claim 2 or Claim 7, characterized in that the drive cable (4; 104) is arranged in the area between the drive unit (9; 109) and the radiation protection container(s) (1; 101) between guiding grooves (14; 114) of the drive or winding drum (13 or 113) and a cover band (16; 116) movable in the driving direction.

10. Radiation therapy device according to one of Claims 1 to 9, characterized in that the radiation protection container(s) (1; 101) is (are) connected to the guiding tubes (12; 112) in such a way that the drive cables (4; 104) can be displaced, with axial guiding, through the guiding tubes (12; 112) and subsequently through the radiation protection container(s) (1; 101) and the flexible tubes (5; 105) located thereafter.

**Revendications**

1. Dispositif de traitement par radiation constitué par:

a) au moins une enceinte (1) de protection contre les radiations (enceinte de repos):

b) des supports d'irradiateurs (2) qui peuvent se déplacer vers l'intérieur et vers l'extérieur dans le sens axial dans un nombre correspondant de conduits souples (5) au moyen d'un nombre correspondant de câbles d'entraînement (4) et

c) un nombre correspondant d'éléments assurant l'accouplement des extrémités libres (8) des conduits souples (5) pour la réalisation de liaisons de passage amovibles avec un nombre correspondant de sondes creuses, caractérisé en ce que

d) tous les câbles d'entraînement (4) sont reliés dans le sens entrée et sortie avec une unité d'entraînement commune (9) et

e) en ce qu'il comprend une autre enceinte (7) de protection contre les radiations (enceinte intermédiaire) qui est associée à un grand nombre égal d'éléments correspondants (6) assurant l'accouplement des conduits souples pour la réalisation de liaisons de passage avec les conduits souples (5) et est disposée de telle manière que tous les conduits souples (5) peuvent lui être reliés d'une manière amovible.

2. Dispositif de traitement par radiation selon la revendication 1, caractérisé en ce qu'il comprend un tambour d'entraînement (13), connu par lui-même, dont le pourtour est plus grand que la longueur d'avance maximale.

3. Dispositif de traitement par radiation selon la revendication 2, caractérisé en ce que les extrémités (disjoncteurs 22) des câbles d'entraînement (4) situées du côté entraînement sont montées de manière à être axialement élastiques dans une barre de commutateur (20).

4. Dispositif de traitement par radiation selon l'une des revendications 1 à 3, caractérisé en ce que la longueur totale de chaque conduit souple (5) et de la sonde creuse qui doit lui être reliée diffère de la longueur totale du même conduit souple (5) et de l'espace libre dans l'enceinte (7) de protection contre les radiations qui doit lui être relié d'au moins le trajet d'enclenchement d'un commutateur terminal.

5. Dispositif de traitement par radiation selon l'une des revendications 1 à 4, caractérisé en ce que les éléments d'accouplement (6) correspondants des conduits souples de l'autre enceinte (7) de protection contre les radiations sont plus écartés que les conduits souples (5) qui sortent de l'autre enceinte (1) de protection contre les radiations.

6. Dispositif de traitement par radiation constitué par:

a) au moins une enceinte (101) de protection contre les radiations;

b) des supports d'irradiateur (102) qui peuvent se déplacer vers l'intérieur et vers l'extérieur dans le sens axial dans un nombre correspondant de conduits souples (105) au moyen d'un nombre correspondant de câbles d'entraînement (104), caractérisé en ce que

c) un câble d'entraînement (104), un support d'irradiateur (102) et une enceinte (101) de protection contre les radiations sont placés ensemble dans une enveloppe en cassette (110) et

d) des enveloppes de cassettes (110) peuvent être montées d'une manière amovible dans le dispositif de traitement par radiation en un nombre librement choisi de telle manière que les câbles d'entraînement (104) des enveloppes de cassette (110) mises en place sont reliés pour l'entraînement à une unité d'entraînement (109) du dispositif de traitement par radiation.

7. Dispositif de traitement par radiation selon la revendication 6, caractérisé en ce qu'il comporte un tambour d'enroulement (113) qui est monté sur l'enveloppe de cassette (110) de manière à pouvoir tourner et sur lequel le câble d'entraînement (104) peut s'enrouler en spirale.

8. Dispositif de traitement par radiation selon la revendication 6, caractérisé en ce qu'il comprend une enveloppe (111) qui est montée sur une enveloppe de cassette (110) et contient une cavité (119) en forme de disque comportant une ouverture de passage (120) ménagée dans une surface frontale (121) pour le câble d'entraînement (104), le côté (a) de la cavité correspondant à peu près à l'épaisseur du câble d'entraînement (104), et dans laquelle le câble d'entraînement (104) peut être poussé et pénétré en formant une spirale.

9. Dispositif de traitement par radiation selon l'une des revendications 2 ou 7, caractérisé en ce que, dans la zone comprise entre l'unité d'entraînement (9, 109) et la ou les (n) enceintes (1, 101) de protection contre les radiations, les câbles d'entraînement (4, 104) sont disposés entre les rainures de guidage (14, 114) du tambour d'entraînement ou d'enroulement (13 ou 113) et une bande couvrante (16, 116) qui peut se déplacer dans le sens de l'entraînement.

10. Dispositif de traitement par radiation selon l'une des revendications 1 à 9, caractérisé en ce que la ou les enceintes (1, 101) de protection contre les radiations sont reliées à des tubes de guidage (12, 112) de telle manière que les câbles d'entraînement (4, 104) peuvent se déplacer en étant guidés axialement dans les tubes de guidage (12, 112), puis dans la ou les enceintes (1, 101) de protection contre les radiations et dans les conduits souples (5, 105) qui leur font suite.

Fig.1

9

# Fig.2

# Fig.5

**Fig. 3**

**Fig. 4**

# Fig. 6

# Fig. 7

Fig.8
(B)

110

IX          IX

Fig.9
(IX-IX)

B          X      110    105        108

111                        102
                           101
                           104
M                          124
122           109

Fig.10
(X-X)

111          119      a

104    120              121

Fig.12
(C)

Fig.11